# EUROPÄISCHE PATENTSCHRIFT

(11) **EP 3 182 101 B1**
(45) Veröffentlichungstag und Bekanntmachung des Hinweises auf die Patenterteilung: **29.01.2025**
(21) Anmeldenummer: 16203283.3
(22) Anmeldetag: 09.12.2016
(51) Int. Cl.: A61M 5/31, G01N 21/90, A61M 5/50, G01B 11/08, G01N 21/958

(54) **SYSTEM ZUR OPTISCHEN ERFASSUNG DER FINGERAUFLAGE EINER GLASSPRITZE**
SYSTEM FOR OPTICAL DETECTION OF THE FLANGE OF A SYRINGE
SYSTÈME DE DÉTECTION OPTIQUE DU FLANC D'UN SERINGUE EN VERRE

(30) Priorität: 14.12.2015 DE 102015121758
(43) Veröffentlichungstag der Anmeldung: 21.06.2017
(73) Patentinhaber: Gerresheimer Bünde GmbH, 32257 Bünde (DE)
(72) Erfinder: Acker, Wolfram, 32257 Bünde (DE); Hellmich, Matthias, 32257 Bünde (DE)
(74) Vertreter: Holz, Christian

(56) Entgegenhaltungen:
- EP-A1- 0 683 388
- EP-A1- 0 764 846
- EP-A1- 2 381 246
- EP-A1- 2 851 677
- DE-A1- 3 237 511
- DE-B4- 102012 008 110

## Beschreibung

Die Erfindung betrifft ein System zur optischen Erfassung einer Fingerauflage einer Glasspritze gemäß dem Oberbegriff des Anspruchs 1 sowie ein Verfahren zur optischen Erfassung einer Fingerauflage einer Glasspritze gemäß dem Oberbegriff des Anspruchs 10.

In der Medizin werden zur Verabreichung von Medikamenten u.a. Spritzen eingesetzt. Diese Spritzen werden normalerweise unbefüllt vom Spritzenhersteller an den Pharmahersteller ausgeliefert, der dann die Befüllung, Verpackung etc. übernimmt. Die Spritzen können jedoch auch beim Spritzenhersteller befüllt und ggfs. verpackt werden, so dass sie als "pre-filled" einsatzbereit an den Pharmahersteller ausgeliefert werden können, d.h. befüllt und mit geschützter Hohlnadel bzw. Kanüle.

Diese Spritzen werden häufig aus durchsichtigem Kunststoff oder durchsichtigem Glas hergestellt. Die Durchsichtigkeit des Materials der Spritze ermöglicht dem verabreichenden Personal eine direkte optische Kontrolle des Inhalts z.B. hinsichtlich Fremdkörper, Ausflockungen, Färbung sowie Füllstand. Ebenso schafft die Durchsichtigkeit des Spritzenkörpers beim Patienten Vertrauen hinsichtlich der an ihm vorzunehmenden Handlung.

Aufgrund seiner chemischen Beständigkeit wird dabei als Material der Spritze vorzugsweise Glas verwendet, weil die Widerstandsfähigkeit des Glases gegen Einwirkungen von Chemikalien eine Korrosion, wie bei Kunststoffen üblich, nahezu vollständig verhindert. Auf diese Weise kann eine Verunreinigung des Medikaments durch das Material der Spritze bestmöglich ausgeschlossen werden. Dabei werden die verschiedenen Glasmaterialien entsprechend ihrer hydrolytischen Resistenz, d.h. dem Maß für die Extrahierbarkeit basischer Verbindungen aus dem Glas durch den Angriff von Wasser bei 98 °C, in verschiedene hydrolytische Klassen eingeteilt. Zur Erfüllung der Anforderungen an die höchste, erste hydrolytische Klasse werden z.B. Borosilikatgläser eingesetzt.

Die Herstellung derartiger Glasspritzen läuft bei einem Glashersteller z.B. üblicherweise wie folgt ab. Von einem zylindrischen Glasrohr wird zunächst ein Stück in einer vorbestimmten Länge abgetrennt, auch Ablängen genannt, welches im Wesentlichen den zylindrischen Spritzenkörper bildet. Ein erstes, vorderes Ende des Spritzenkörpers wird erhitzt und durch Verengen seines Durchmessers zu einer Bohrung verformt, in die später die Hohlnadel (Kanüle) z.B. eingeklebt werden kann. Der Spritzenkörper an sich bleibt in seiner Form und in seinem Durchmesser erhalten und entspricht etwa dem Durchmesser des Kolbens, der sich bei Gebrauch innerhalb des Spritzenkörpers in Längsrichtung der Glasspritze bewegen können soll.

Das zweite, hintere Ende des Spritzenkörpers, welches der Bohrung diametral gegenüberliegt, bleibt in seinem Durchmesser erhalten, um hier später den Kolben der Spritze aufnehmen zu können. Durch Erhitzen des Materials wird dieses zweite, hintere Ende des Spritzenkörpers zumindest teilweise radial flächig aufgeweitet, so dass hier eine so genannte Fingerauflage zur Handhabung der Spritze ausgebildet werden kann. Bei der Handhabung der Spritze fassen ein Zeige- und ein Ringfinger des Benutzers von der Seite des Spritzenkörpers hinter die Fingerauflage und der Daumen drückt den Kolben in der entgegengesetzten Richtung in den Spritzenkörper hinein, wodurch der Spritzeninhalt über die Hohlnadel der Bohrung aus dem Spritzenkörper heraus abgegeben wird. Diese Fingerauflage ist üblicherweise zylindrisch ringförmig geschlossen oder auch zylindrisch ringförmig unterbrochen mit zwei radial hervorragenden Abschnitten ausgebildet.

Ist der eigentliche Spritzenkörper aus Glas fertig hergestellt, so kann dieser optional mit einer Beschriftung z.B. in Form einer Füllstandskala versehen werden. Anschließend wird die Hohlnadel in die Bohrung eingeklebt, wobei auch das Aufsetzen einer Luer-Verbindung bzw. Luer-ähnlichen Verbindung auf bzw. in die Bohrung möglich ist. Die Glasspritze wird dann gewaschen und sterilisiert, um Fremdkörper und Verunreinigungen zu entfernen. Die Hohlnadel wird anschließend z.B. mit einer Kunststoffkappe verschlossen und gegen äußere Einwirkungen geschützt. Mit der Hohlnadel nach unten zeigend wird der Spritzenkörper dann vom anderen Ende her befüllt und von dieser Seite mit dem Kolben verschlossen. Damit ist die Glasspritze einsatzbereit und sie kann verpackt werden.

Bei diesem Herstellprozess werden sehr hohe Anforderungen an die Qualität der Glasspritze gestellt, weil deren Beschaffenheit einen entscheidenden Einfluss auf die folgenden Abfüllungsschritte sowie die Verwendung beim Patienten hat. So können abweichende Maße der Glasspritze zu Montagefehlern oder Undichtigkeiten z.B. beim Einkleben der Hohlnadel oder beim Einfügen des Kolbens führen. Zu dünne Wandstärken des Spritzenkörpers sowie Kratzer oder Risse im Spritzenkörper oder in der Fingerauflage können in der Herstellung oder bei der Anwendung zum Splittern sowie zur Unsterilität der Glasspritze führen. Eine zu dünne Fingerauflage kann bei der Anwendung abbrechen. Diese Gefahren bestehen insbesondere deshalb, weil das Glas als Spritzenmaterial auf seine chemische Beständigkeit und nicht mechanische Stabilität hin optimiert angewendet wird.

Werden diese Mängel vor dem folgenden Abfüllungsschritt nicht erkannt, kann es dort z.B. zum Absplittern eines Teils der Glasspritze oder zum vollkommenen Zerbersten der Glasspritze kommen, wodurch es aufgrund von Glasteilen in den Produktionsanlagen zu Produktionsausfällen kommen kann. Kommt es hierdurch zu Undichtigkeiten, so kann die Sterilität des Medikaments zunichte gemacht werden. Werden die Mängel erst beim Anwender bzw. Patienten aber vor der Benutzung erkannt, wird sehr wahrscheinlich ein hoher Vertrauensverlust des Patienten zum verabreichenden Personal sowie seinerseits vom Anwender gegenüber dem Lieferanten der defekten Glasspritze, d.h. dem Pharmahersteller, eintreten. Beides kann zu einem Wechsel des Arztes durch den Patienten bzw. des Pharmalieferanten durch den Arzt oder das Krankenhaus führen. Ganz besonders aber sind derartige Vorfälle für den Pharmahersteller höchst relevant, weil sie zu einer Rückrufaktion des betroffenen Produktes sowie zu einem Verlust der entsprechenden Produktzulassung führen können (Good manufacturing practice). Daher wird seitens des Pharmaherstellers bzw. des Herstellers von Glasspritzen der größtmögliche Maßstab an Qualität an die Glasspritzen gelegt, um jegliche Mängel mit an Sicherheit grenzender Wahrscheinlichkeit ausschließen zu können.

Die Spritzenkörper werden daher üblicherweise sowohl auf dimensionale Aspekte, d.h. geometrische Merkmale, als auch auf kosmetische Aspekte überprüft. Bei einem Spritzenkörper können dimensionale Aspekte z.B. der Durchmesser des zylindrischen Spritzenkörpers, der Durchmesser der Bohrung zum Aufsetzen der Hohlnadel (Kanüle) mittels Luer-Verbindung bzw. Luer-ähnlicher Verbindung, die Schulterhöhe des Spritzenkörpers in Längsrichtung, die Halslänge des Spritzenkörpers in Längsrichtung, der Durchmesser des Endes zur Kolbenaufnahme, die Längenmessung des Spritzenkörpers als Ganzes in Längsrichtung, die Exzentrizität des Spritzenkörpers bzw. der Bohrung gegenüber der Längsachse, die Mündungsplanheit des Endes der Fingerauflage bzw. des Endes zur Kolbenaufnahme etc. sein. Unter kosmetischen Aspekten können Verschmutzungen, Fremdkörper, Kratzer, Narben, Risse, abgeplatzte Stellen, Einschlüsse oder auch sonstige kundenspezifische Merkmale verstanden werden.

Speziell bei der Fingerauflage des Spritzenkörpers aus Glas können als dimensionale Fehler Abweichungen der kreisförmigen Umfangsform bzw. Ausbildung der beiden radial hervorragenden Abschnitte und abweichende bzw. ungenügende Stärken (Dicke in Längsrichtung) des Glasmaterials, d.h. sogenannte Einfallstellen, sowie als kosmetische Fehler Kratzer, Narben, Risse, Einschlüsse und abgeplatzte Stellen auftreten.

Dem Anwender können die dimensionalen Fehler sowohl haptisch als auch optisch dadurch auffallen, da sie die Handhabung beeinträchtigen. Auch kann die Fingerauflage bei der Anwendung abbrechen. Die kosmetischen Fehler können direkt oder durch das Abbrechen der Fingerauflage als Ganzes oder als Teilstück oder das Zerbrechen der Glasspritze als Ganzes zu Verletzungen des Anwenders führen. Ferner können bei der Herstellung Verschmutzungen auftreten, die an dem übrigen Glaskörper anhaften oder auf bzw. in diesem liegen bleiben. Diese können zwar teilweise durch den Waschprozess entfernt werden, jedoch nicht sicher vollständig. Ferner sind Verschmutzungen als anorganische Schadstoffe durch die Sterilisation nicht betroffen. Diese können, falls sie sich auf der Fingerauflage befinden, durch das Aufrichten der Glasspritze mit der Spitze nach unten zum Befüllen des Inhalts in den Spritzenkörper hinein gelangen.

Bei der Herstellung sind Risse der Fingerauflage sowie Einfallstellen der Fingerauflage ferner ein häufiger Grund für den Bruch der Glasspritze während der Produktion beim anschließenden Waschprozess oder bei der Abfüllung. Daher kommt der Inspektion der Fingerauflage einer Glasspritze eine besondere Bedeutung bei der Herstellung von Glasspritzen zu.

Zur Qualitätskontrolle in der Produktion sind generell optische Verfahren z.B. mittels Kameratechnik bekannt, bei denen Signale verarbeitet werden, die Bilder repräsentieren (Bildverarbeitung). Diese optischen Verfahren werden auch zur Kontrolle von Glasprodukten eingesetzt, wobei sich durch die Durchsichtigkeit des Glasmaterials besondere Herausforderungen an die Bilderfassung sowie die anschließende Bildverarbeitung ergeben.

Zur Kontrolle der Fingerauflage eines Spritzenkörpers aus Glas sowohl nach dimensionalen als auch nach kosmetischen Aspekten ist es bekannt, den Spritzenkörper auf einer Auflagefläche einer Handhabungseinheit zu positionieren und ggfs. dort zu fixieren. Mittels einer Flächenkamera wird die Fingerauflage direkt optisch erfasst, d.h. die Flächenkamera ist direkt auf die Fingerauflage hin gerichtet und erfasst diese als zweidimensionales Bild. Unter einer Flächenkamera wird dabei eine Kamera verstanden, welche einen zweidimensionalen Sensor besitzt, d.h. das zu erfassende Objekt flächig in einer X-Y-Ebene erfasst, die sich aus einer X-Richtung sowie einer hierzu senkrechten Y-Richtung ergibt.

Die Ausleuchtung des Spritzenkörpers erfolgt üblicherweise von der gegenüberliegenden Rückseite der Glasspritze, d.h. von der Seite der Bohrung des Spritzenkörpers und somit von der Seite der Fingerauflage, hinter welche die Finger bei der Benutzung greifen. Somit erfolgt die Ausleuchtung der Fingerauflage von der der Flächenkamera abgewandten Seite am Spritzenkörper seitlich vorbei bzw. durch den Spritzenkörper hindurch. Die Lichtquelle der Ausleuchtung ist üblicherweise flächig ausgebildet und entspricht in ihrer Fläche im Allgemeinen etwa der Fläche des auszuleuchtenden Objekts, um Bauraum für das Bildverarbeitungssystem zu sparen. Aus den gleichen Gründen sind sowohl die Flächenkamera als auch die Lichtquellen seitlich möglichst nah an dem zu erfassenden Spritzenkörper angeordnet.

Nachteilig ist hierbei, dass die Lichtquelle aufgrund ihres etwa der Fläche des auszuleuchtenden Objekts entsprechenden Durchmessers nahezu ideal parallele Lichtstrahlen zum Spritzenkörper hin aussendet. Hierdurch wird ein Großteil der Lichtstrahlen durch die Bohrung, den Spritzenkörper und die Fingerauflage durch Brechung an den Glasoberflächen soweit abgelenkt, dass nur sehr wenig Licht von dem Objektiv der Flächenkamera aufgefangen werden kann. Daher werden durch die Flächenkamera eher große schwarze Kanten bzw. Flächen erfasst bzw. ergeben sich große Kontraste, von denen kein gebrochenes Licht das Objektiv erreicht und die eine Bestimmung von dimensionalen und kosmetischen Merkmalen nur in unzureichender Qualität für den beschriebenen Anwendungsfall erlauben. Ferner befindet sich die Auflagefläche des Spritzenkörpers sowie deren Halterung, die beides Bestandteile einer Handhabungseinheit zur Führung und Positionierung des Spritzenkörpers während der Inspektion der Fingerauflage sein können, zwischen der Lichtquelle und der Flächenkamera, wodurch es zum einen durch diese Körper zu einer Schattenbildung kommt, die ebenfalls die Qualität der Bilderfassung beeinträchtigt, und zum anderen die Auflagefläche samt Halterung selbst vom Bild der Flächenkamera teilweise miterfasst wird.

Zur Verbesserung der Qualität der Bilderfassung für das zuvor beschriebene System wäre eine deutliche Vergrößerung der Lichtquelle und ein deutlich größerer Abstand zwischen Lichtquelle und Glasspritze erforderlich, so dass deutlich mehr Lichtstrahlen insgesamt sowie aus anderen Winkeln auf den Spritzenkörper treffen und trotz der zuvor beschriebenen Brechungen auch das Objektiv der Flächenkamera erreichen würden. Hierdurch würden die schwarzen Kanten kleiner und damit das erfasste Bild schärfer werden.

Dies ist in der Praxis jedoch nicht umsetzbar, weil sich neben der Verteuerung des Bilderfassungssystems an sich hierdurch der Bauraum des Bilderfassungssystems entsprechend vergrößern würde. Dies führt zu einer Vergrößerung der Produktionsanlage als Ganzes, was aufgrund der damit verbundenen Kosten höchst unerwünscht ist.

Ferner wäre die Auflagefläche samt Halterung der Handhabungseinheit dann weiter störend im erfassten Bild zu sehen und würde ebenfalls weiterhin einen Schatten erzeugen. Dies würde wiederum die Qualität der Bilderfassung beeinträchtigen und damit die über die Vergrößerung der Lichtquelle erreichte Verbesserung zumindest teilweise wieder aufheben. Außerdem ist der Schattenwurf der Auflagefläche samt Halterung umso größer, desto größer die Lichtquelle bzw. deren Ausleuchtung von der der Flächenkamera gegenüberliegenden Seite ist; dies spricht gegen eine Vergrößerung der Lichtquelle bei einem derartigen Bilderfassungssystem.

Alternativ ist es bekannt, die Ausleuchtung der Glasspritze von der gleichen Seite wie die Bilderfassung mit der Flächenkamera vorzunehmen. Hierzu können die Lichtquellen der Ausleuchtung neben der Flächenkamera bzw. um die Flächenkamera herum angeordnet werden, so dass sie direkt von der Seite auf die Fingerauflage leuchten, von der die Fingerauflage durch die Flächenkamera erfasst wird. Um Objekte wie die Auflagefläche des Spritzenkörpers samt Halterung der Handhabungseinheit im Hintergrund von der Aufnahme durch die Flächenkamera auszuschließen, kann bei diesem System eine Verblendung um den Spritzenkörpers herum vorgenommen werden. Diese Verblendung besteht aus zwei Halbschalen mit Aussparungen für den Spritzenkörper, die im Bereich zwischen der Fingerauflage und der Auflage an den Spritzenkörper herangefahren werden und miteinander abschließen.

Nachteilig ist hierbei zum einen, dass diese Verblendung als zusätzliche Maßnahme vorgenommen und produktspezifisch hergestellt werden muss. Ferner lässt sich ein Spalt an der Stelle, an der sich die beiden Halbschalen zur Verblendung schließen, nicht vollständig vermeiden. Auch bleibt stets ein Spalt zwischen der Verblendung und dem Spritzenkörper. Diese Spalten können von der Flächenkamera durch die Fingerauflage hindurch erfasst werden und das erfasste Bild stören. Des Weiteren kann es an der Verblendung zu Reflexionen kommen, die auch durch die zu erfassende Fingerauflage hindurch zur Flächenkamera gelangen und die Bilderfassung verschlechtern können.

Die EP 0 764 846 A1 beschreibt ein System zur Inspektion von Behältern, insbesondere von Glasflaschen. Ein Behälter wird in stationärer Position auf seinem Boden stehend gehalten und um seine Hochachse gedreht. Lichtenergie wird von unten auf den Boden des Behälters gerichtet, wenn dieser gedreht wird, und eine Kamera erhält von oben durch den Flaschenhals des Behälters hindurch ein lineares oder ein-dimensionales Bild des Bodens des Behälters, das von der Lichtenergie beleuchtet wird. Das Behälterbild, das aus einer Vielzahl von Pixeldatenbytes besteht, die jeweils einen Teil des Bildes bilden, wird seriell und nacheinander zu einer programmierbaren Logikeinrichtung geleitet, in der ein Array von Hardware-Logikelementen so konfiguriert ist, dass die Elemente des Arrays arbeiten auf einer Vielzahl von aufeinanderfolgenden Pixeldatenbytes gleichzeitig und in Echtzeit. Die Pixeldaten werden analysiert, um Inspektionsinformationen als eine Funktion der optischen Eigenschaften des Behälters zu erhalten.

Die EP 2 381 246 A1 beschreibt eine Vorrichtung zum Prüfen der Oberfläche eines lichtdurchlässigen Gegenstandes wie z.B. eine Glasspritze mit einer Kante mit einer Endfläche. Die Vorrichtung weist einen Träger zum Tragen des Gegenstandes entlang seiner Hochachse auf, welcher um die Hochachse gedreht werden kann. Innerhalb des Trägers ist eine Lichtquelle vorgesehen, die ein Licht in einer Richtung senkrecht zur Hochachse emittieren kann. Die Lichtquelle wird durch den Träger möglichst vollständig eingeschlossen, so dass das Licht in eine Kante des Gegenstands eingeleitet und durch diesen hindurch entlang seiner Hochachse geführt werden kann. Dank des in den Gegenstand eingeleiteten Lichts können kosmetische Fehler mit dem menschlichen Auge besser erkannt werden. Dies kann auch mit einer Flächenkamera durchgeführt werden, welche senkrecht zur Hochachse auf den Gegenstand gerichtet wird. Dabei kann jeweils ein kleiner Winkelbereich der zylindrischen Oberfläche des sich drehenden Gegenstands erfasst werden, so dass die Krümmung des Gegenstands nicht stört.

Die EP 0 683 388 A1 beschreibt, dass eine Spritze beispielsweise von der Seite mit Licht beleuchtet wird. Die Bildverarbeitung wird mit einem Bildsignal durchgeführt, das durch Abbilden eines von der Kante eines Flansches der Spritze reflektierten Lichts mittels einer TV-Kamera oder einer CCD-Kamera von schräg oben erfasst wird. Durch Erfassen eines unregelmäßigen Zustands des Bildsignals, der durch das reflektierte Licht verursacht wird, wird ein Riß oder Chip in dem Flansch erkannt. Der unregelmäßige Zustand des Bildsignals, der durch Detektieren von Teilen erhalten wird, von denen Licht nicht reflektiert wird, liefert ein Verfahren, mit dem es leicht ist, einen Riß oder Chip in dem Flansch zu erkennen.

Die Aufgabe der vorliegenden Erfindung ist es, ein System zur optischen Erfassung der Fingerauflage einer Glasspritze der eingangs beschriebenen Art bereit zu stellen, so dass die Bilderfassung, insbesondere von dimensionalen und bzw. oder kosmetischen Merkmalen, bei kompakter Bauweise des Systems verbessert werden kann.

Die Aufgabe wird erfindungsgemäß durch die Merkmale des Anspruchs 1 sowie des Anspruchs 10 gelöst. Vorteilhafte Weiterbildungen sind in den Unteransprüchen beschrieben.

Somit betrifft die vorliegende Erfindung, wie in Anspruch 1 definiert, ein System zur optischen Erfassung einer Fingerauflage einer Glasspritze mit einer Glasspritze, welche entlang ihrer Längsachse einen zylindrischen Spritzenkörper mit einem vorderen Ende mit einer Bohrung und mit einem hinteren Ende mit einer Fingerauflage aufweist, und mit einer optischen Bilderfassungseinheit zum Erfassen des Untersuchungsbereichs des Glaskörpers. Die optische Bilderfassungseinheit weist eine Zeilenkamera mit einem zeilenförmigen Erfassungsbereich auf. Das System weist ferner eine Bewegungseinheit auf, welche eingerichtet ist, die Glasspritze und die Zeilenkamera relativ zueinander derart zu bewegen, so dass die Fingerauflage einer Glasspritze über den Erfassungsbereich der Zeilenkamera, oder umgekehrt, geführt wird.

Unter einer Zeilenkamera wird dabei ein Kameratyp verstanden, der nur eine lichtempfindliche Zeile, d.h. einen Zeilensensor aufweist. Im Gegensatz hierzu weisen Flächenkameras einen zweidimensionalen Sensor auf, der über eine Vielzahl von Zeilen verfügt (Flächensensoren). Um mit einer Zeilenkamera dennoch ein zweidimensionales Bild erzeugen zu können, ist es erforderlich, die zu erfassende Glasspritze und die aufnehmende Zeilenkamera relativ zueinander zu bewegen, um die einzelnen nacheinander erfassten Zeilenbilder zu dem zweidimensionalen Bild aneinander setzen zu können. Hierbei kann sowohl die Zeilenkamera mit ihrem Erfassungsbereich über die Fingerauflage der stillstehenden Glasspritze geführt werden oder die Zeilenkamera steht still und die Glasspritze wird relativ zu ihr bewegt.

Der vorliegenden Erfindung liegt dabei die Erkenntnis zugrunde, dass aufgrund des schmalen Erfassungsbereichs der Zeilenkamera die Bilderfassung störender Objekte wie z.B. einer Auflagefläche, deren Halterung oder anderer Körper einer Handhabung- bzw. Führungseinheit der Glasspritze, welche bei einer Flächenkamera mit erfasst werden würden, vollständig vermieden werden kann. Mit anderen Worten können die Effekte der Körper der Handhabungseinheit, welche stets erforderlich sind, um die Glasspritze während der Inspektion der Fingerauflage zu halten und zu positionieren, durch die Bilderfassung mittels Zeilenkamera vermieden werden, weil die Handhabungseinheit durch die zeilenförmige Erfassung der Fingerauflage vollkommen von der Bilderfassung ausgeschlossen werden kann. Dies kann die Qualität der Bilderfassung verbessern, weil sichergestellt werden kann, dass ausschließlich der relevante Untersuchungsbereich der Glasspritze erfasst wird.

Gemäß einem weiteren Aspekt der vorliegenden Erfindung ist die Bewegungseinheit eingerichtet, die Glasspritze relativ zur Zeilenkamera, oder umgekehrt, derart um eine Längsachse zu drehen, so dass die Fingerauflage der Glasspritze über den Erfassungsbereich der Zeilenkamera, oder umgekehrt, geführt wird. Dies kann eine kompakte Relativbewegung von Glasspritze und Zeilenkamera zueinander ermöglichen.

Gemäß einem weiteren Aspekt der vorliegenden Erfindung ist die Fingerauflage der Glasspritze oder der Erfassungsbereich der Zeilenkamera senkrecht zur Längsachse ausgerichtet. Die Längsachse bildet dabei die Rotationsachse der Relativbewegung zwischen Glasspritze und Zeilenkamera. Hierdurch kann ein Bild der Ebene der Glasspritze senkrecht zur Längsachse bzw. zur Rotationsachse erfasst werden. Bei einem zylindrischen Glaskörper wie der Glasspritze kann auf diese Art und Weise der vordere Bereich der Bohrung oder der hintere Bereich der Fingerauflage flächig erfasst werden. Durch die Bilderfassung mittels Zeilenkamera und die rotatorische Relativbewegung der Glasspritze ergibt sich hierdurch zwar nicht das gewohnte optische Bild einer Flächenkamera sondern eine verzerrte Darstellung der Oberfläche der Glasspritze in dieser Ebene. Jedoch ist diese Darstellung durch die Verwendung des Zeilensensors in einer weitaus höheren Auflösung und Qualität möglich als bei bisherigen Flächenkameras. Ferner kann auch diese verzerrte Darstellung z.B. für einen Soll-Ist-Vergleich entsprechend aufbereiteter Soll-Konturen der Glasspritze verwendet werden, um dimensionale Fehler erkennen zu können. Auch lassen sich in dieser Darstellung kosmetische Fehler erkennen. Des Weiteren können Bereiche der Handhabungseinheit in dieser Bilddarstellung nicht vorhanden sein, weil sie durch die Verwendung einer Zeilenkamera gar nicht erfasst wurden.

Gemäß der beanspruchten Erfindung ist das System dazu eingerichtet, die Glasspritze relativ zur Zeilenkamera zu bewegen.

Mit anderen Worten ist die Zeilenkamera fest angeordnet und steht still. Die Glasspritze kann an der Zeilenkamera derart vorbeigeführt werden, dass ihre Fingerauflage über den Erfassungsbereich der Zeilenkamera geführt wird. Dies ist vorteilhaft, weil die Flächenkamera im Allgemeinen das größere und schwerere Objekt im Vergleich zur Glasspritze ist und damit ihre Bewegung relativ zur Glasspritze aufwändiger wäre. Ferner kann die Ausrichtung von Fingerauflage und Erfassungsbereich zueinander auf diese Weise einfacher sein, wenn die leichtere und kleinere Glasspritze bewegt wird. Dies kann dadurch auch die Qualität der Bilderfassung verbessern. Auch kann sich das erfindungsgemäße System so einfacher in einer Glasproduktionsanlage der Massenproduktion einbinden lassen, in der die Glasspritzen ohnehin z.B. getaktet durch die verschiedenen Produktionsstationen hindurch bewegt werden.

Gemäß einem weiteren Aspekt der vorliegenden Erfindung weist das System ferner eine Beleuchtungseinheit zur Ausleuchtung wenigstens der Fingerauflage der Glasspritze auf. Diese Beleuchtungseinheit kann dadurch die Qualität der Bilderfassung verbessern, dass sie die Helligkeit der Fingerauflage der Glasspritze erhöht. Auf diese Weise können mehr Lichtstrahlen von der Fingerauflage zur Zeilenkamera gelangen und von dessen Zeilensensor erfasst werden. Hierdurch können die Kanten der Glasspritze schärfer werden. Dies kann sowohl für dimensionale als auch kosmetische Aspekte gelten.

Die Beleuchtungseinheit kann eine einzelne Lichtquelle sein oder auch eine Mehrzahl einzelner Lichtquellen aufweisen, die als Beleuchtungseinheit als Ganzes zusammenwirken. Vorzugsweise werden als Lichtquellen eine Vielzahl von LEDs (Light Emitting Diode) eingesetzt, die möglichst gleichmäßig strukturiert und so zueinander beabstandet angeordnet werden, dass sie eine möglichst diffuse und homogene Beleuchtung erzeugen können.

Gemäß einem weiteren Aspekt der vorliegenden Erfindung weist die Beleuchtungseinheit einen geringeren Abstand zu der Fingerauflage der Glasspritze als die Bilderfassungseinheit auf. Auf diese Weise kann das Licht maximal nah an die zu erfassende Glasspritze bzw. dessen Fingerauflage heran gebracht werden. Dies kann die Lichtausbeute, d.h. die Menge der Lichtstrahlen, die zur Bilderfassung verfügbar sind, und damit auch die Qualität der Bilderfassung steigern.

Gemäß einem weiteren Aspekt der vorliegenden Erfindung weist die Beleuchtungseinheit einen Abstand von weniger als 10 mm zu der Fingerauflage des Glasspritze auf. Bis zu diesem Grenzwert kann die Lichtausbeute bei der Bilderfassung von Glasspritzen groß genug sein, um dimensionale und kosmetische Merkmale der Glasspritze mit ausreichend hoher Qualität erkennen zu können.

Gemäß einem weiteren Aspekt der vorliegenden Erfindung ist die Beleuchtungseinheit eingerichtet, eine im Wesentlichen rechteckige Ausleuchtung der Fingerauflage der Glasspritze zu erzeugen, welche wenigstens dem Erfassungsbereich der Zeilenkamera entspricht. Die Zweckmäßigkeit der rechteckigen Ausleuchtung der Fingerauflage ergibt sich aus dem zeilenförmigen Erfassungsbereich der Zeilenkamera, welche aufgrund seiner Zeilenförmigkeit ebenfalls als rechteckig anzusehen ist. Um diese Zeile vollständig und gleichmäßig ausleuchten zu können, kann eine Beleuchtungseinheit ausreichend sein, welche im Wesentlichen dieser Form entspricht, d.h. eine geringe Höhe aber große Breite aufweist, so dass sie den Zeilensensor vollständig ausleuchten kann, aber nicht wesentlich über dessen Erstreckung hinausgeht. Eine derartige Beleuchtungseinheit kann somit aus einer flachen aber breiten und durchgehenden Lichtquelle gebildet werden, so dass sich keine dunkleren Zwischenbereiche zwischen den einzelnen Lichtquellen bilden, die zu einer Inhomogenität der Ausleuchtung führen könnten. Wird die Beleuchtungseinheit aus einzelnen punktförmigen Lichtquellen wie z.B. LEDs gebildet, so können diese dennoch dichter in der breiten Erstreckung der Zeilenkamera positioniert werden und so die Homogenität ihrer Ausleuchtung verbessern als bei einer flächigen Beleuchtungsanordnung, weil die einzelnen LEDs von den zwei verbleibenden Seiten besser zugänglich sein können als bei einer flächigen Anordnung von LEDs.

Erfindungsgemäß weist der Glaskörper wenigstens einen weiteren Bereich auf, welcher zwischen der Fingerauflage der Glasspritze und der Bilderfassungseinheit angeordnet ist. Auf diese Weise kann dieser weitere Bereich von der Beleuchtungseinheit abgewandt angeordnet werden, so dass die Beleuchtungsanordnung näher an der Fingerauflage der Glasspritze positioniert werden kann. Dies kann die Ausleuchtung der Fingerauflage und damit die Qualität der Bilderfassung verbessern.

Erfindungsgemäß ist die Bilderfassungseinheit eingerichtet, die Fingerauflage der Glasspritze außen an dem Spritzenkörper der Glasspritze entlang der Längsachse vorbei zu erfassen. Mit anderen Worten kann die Zeilenkamera die Fingerauflage der Glasspritze an zumindest einem Teil des Glaskörpers vorbei erfassen. Bei einer Glasspritze können dies z.B. der Spritzenkörper und die Bohrung sein. Hierbei kann in Kauf genommen werden, dass Teile der Fingerauflage durch die weiteren Bereiche wie z.B. den Spritzenkörper zumindest teilweise verdeckt werden. Diese Anordnung kann es jedoch ermöglichen, die Beleuchtungseinheit möglichst nah an der Fingerauflage zu positionieren und hierdurch die Lichtausbeute zu maximieren. Hierdurch kann sich die Qualität der Bilderfassung steigern, auch wenn die Fingerauflage durch die Zeilenkamera von weiter weg und an der Glasspritze teilweise vorbei erfasst werden muss. Dabei kann die Erfassung der Fingerauflage durch die Bilderfassungseinheit sowohl direkt, d.h. ohne Umlenkung des Strahlengangs, oder indirekt, d.h. mit Umlenkung der Strahlengang z.B. über Spiegel, erfolgen. Letztere Anordnung hat den Vorteil, dass bei der Anordnung der Komponenten des erfindungsgemäßen Bilderfassungssystems weitere Freiheiten geschaffen werden. Ferner kann hierdurch u.U. ein kompakterer Aufbau des Bilderfassungssystems erreicht werden.

Erfindungsgemäß weist die Bilderfassungseinheit ein Objektiv mit einer derart großen Brennweite auf, so dass Lichtstrahlen von der Fingerauflage der Glasspritze etwa parallel vom Objektiv erfasst werden können. Hierunter ist eine fast telezentrische Brennweite zu verstehen, bei der die Lichtstrahlen quasi ideal parallel zueinander verlaufen, d.h. der Brennpunkt etwa im Unendlichen liegt. Eine derart große Brennweite kann der Zeilenkamera die Erfassung der Fingerauflage der Glasspritze an dem Spritzenkörper der Glasspritze vorbei ermöglichen, d.h. über diese hinweg, welche sonst die Fingerauflage insbesondere durch ihre Kanten zumindest teilweise verdecken könnten.

Erfindungsgemäß weist das Objektiv eine telezentrische Brennweite oder eine hyperzentrische Brennweite auf. Bei der telezentrischen Brennweite liegt der Brennpunkt im Unendlichen und die Lichtstrahlen verlaufen ideal parallel zueinander. Bei der hyperzentrischen Brennweite werden weiter entfernte, aber gleich große Objekte in der Bildebene größer dargestellt als nähere. In beiden Fällen kann sichergestellt werden, dass der Spritzenkörper der Glasspritze ausschließlich direkt hinter ihm liegende Bereiche der Fingerauflage verdecken kann, jedoch an ihm durch die Bilderfassungseinheit sicher parallel vorbeigeschaut werden kann.

Gemäß einem weiteren Aspekt der vorliegenden Erfindung weist die Bewegungseinheit eine Antriebseinheit auf, die eingerichtet ist, die Glasspritze relativ zur Zeilenkamera, oder umgekehrt, zu bewegen. Hierdurch kann die nötige Relativbewegung zwischen Fingerauflage und Erfassungsbereich durch das Bilderfassungssystem selber direkt umgesetzt werden. Vorzugsweise wird die Glasspritze durch die Bewegungseinheit relativ zur Zeilenkamera bewegt. Hierdurch kann der Aufwand der Relativbewegung minimiert werden, da im Allgemeinen die Glasspritze kleiner und leichter als die Zeilenkamera ist.

Gemäß einem weiteren Aspekt der vorliegenden Erfindung weist das System ferner eine Führungseinheit auf, welche eingerichtet ist, die Glasspritze bei einer Relativbewegung zur Zeilenkamera, oder umgekehrt, zu führen. Die Relativbewegung kann vorzugsweise rotatorisch erfolgen, da die Glasspritze ein zylindrischer Körper, wie insbesondere ein Spritzenkörper, ist. Hierdurch kann die Handhabung bzw. Bewegung der Glasspritze bzw. der Zeilenkamera unterstützt werden, weil dieser bzw. diese durch die Führungseinheit während der Relativbewegung gehalten werden kann. Dies kann dahingehend besonders wichtig sein, weil während der Relativbewegung der Fingerauflage und der Erfassungsbereich reproduzierbar und vorbestimmter zueinander in Deckung gebracht werden müssen.

Die vorliegende Erfindung betrifft, wie in Anspruch 10 definiert, auch ein Verfahren zur optischen Erfassung einer Fingerauflage einer Glasspritze. Die Einzelheiten und Vorzüge des erfindungsgemäßen Verfahrens geben sich aus dem zuvor beschriebenen erfindungsgemäßen System.

Ein Ausführungsbeispiel und weitere Vorteile der Erfindung werden nachstehend im Zusammenhang mit den folgenden Figuren erläutert. Darin zeigt:
- Fig. 1: eine schematische Schnittdarstellung durch einen Glaskörper in Form einer Glasspritze entlang der Längsachse;
- Fig. 2a: eine schematische Draufsicht auf einen zylindrischen Untersuchungsbereich einer Glasspritze senkrecht zur Längsachse;
- Fig. 2b: eine schematische Draufsicht auf einen zylindrisch unterbrochenen Untersuchungsbereich einer Glasspritze senkrecht zur Längsachse;
- Fig. 3: eine schematische Schnittdarstellung durch ein Bilderfassungssystem für Glaskörper in Form von Glasspritzen entlang der Längsachse gemäß dem Stand der Technik;
- Fig. 4a: eine schematische Darstellung eines flächigen Erfassungsbereichs eines zylindrischen Untersuchungsbereichs senkrecht zur Längsachse gemäß dem Stand der Technik;
- Fig. 4b: eine schematische Darstellung eines flächigen Erfassungsbereichs eines zylindrisch unterbrochenen Untersuchungsbereichs senkrecht zur Längsachse gemäß dem Stand der Technik;
- Fig. 5a: eine schematische Schnittdarstellung durch ein erfindungsgemäßes Bilderfassungssystem für Glaskörper in Form von Glasspritzen entlang der Längsachse von der Seite;
- Fig. 5b: eine schematische Schnittdarstellung durch ein erfindungsgemäßes Bilderfassungssystem für Glaskörper in Form von Glasspritzen entlang der Längsachse von Oben;
- Fig. 6a: eine schematische Darstellung eines zeilenförmigen Erfassungsbereichs eines zylindrischen Untersuchungsbereichs senkrecht zur Längsachse gemäß der vorliegenden Erfindung; und
- Fig. 6b: eine schematische Darstellung eines zeilenförmigen Erfassungsbereichs eines zylindrisch unterbrochenen Untersuchungsbereichs senkrecht zur Längsachse gemäß der vorliegenden Erfindung.

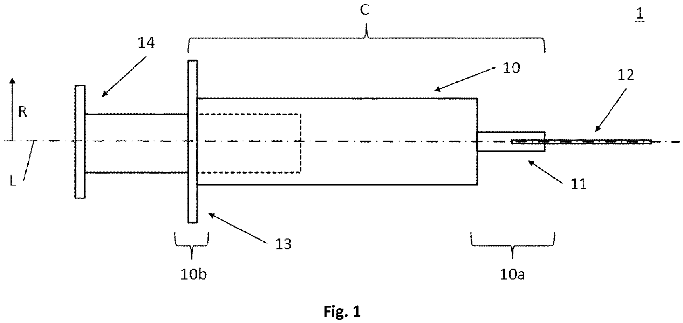

Fig. 1 zeigt eine schematische Schnittdarstellung durch einen Glaskörper 1 in Form einer Glasspritze 1 entlang der Längsachse L. Die Glasspritze 1 weist einen Spritzenkörpers 10 auf, der zylindrisch und rotationsymmetrisch zur Längsachse L ausgebildet ist. Der Spritzenkörpers 10 weist ein erstes, vorderes Ende 10a auf, an dem einstückig mit dem Spritzenkörper 10 eine Bohrung 11 ausgeformt ist, welche eine Hohlnadel 12 oder Kanüle 12 aufnimmt. Der Spritzenkörpers 10 weist ferner ein zweites, hinteres Ende 10b auf, an dem einstückig eine Fingerauflage 13 in Form eines in radialer Richtung R hervorragenden Abschnitts ausgebildet ist, der zylindrisch geschlossen oder unterbrochen ausgebildet sein kann. Ein Kolben 14 ragt zumindest teilweise vom zweiten, hinteren Ende 10b des Spritzenkörpers 10 in dessen hohlen Innenraum hinein, indem z.B. das über die Hohlnadel 12 zu verabreichenden Medikament enthalten sein kann. Die Glasspritze 1 besitzt von der in Längsrichtung L äußeren Seite der Fingerauflage 13 bis zum vorderen, äußeren Ende der Bohrung 11 eine Gesamtlänge C.

Eine derartige Glasspritze 1 und insbesondere deren Fingerauflage 13 soll im Rahmen einer automatisierten Produktion auf ihre Qualität mittels Bilderfassung und anschließender Bildverarbeitung überprüft werden. Hierzu soll nach Fertigstellung der Glasspritze 1 selbst, d.h. ohne eingeführten Kolben 14, eingeklebte Hohlnadel 12 und eingefülltes Medikament, die Bilderfassung erfolgen. Insbesondere soll die Fingerauflage 13 auf dimensionale und kosmetische Merkmale untersucht werden. Somit stellt die Fingerauflage 13 der Glasspritze 1 dem zu erfassenden Untersuchungsbereich 13 des Glaskörpers 1 dar.

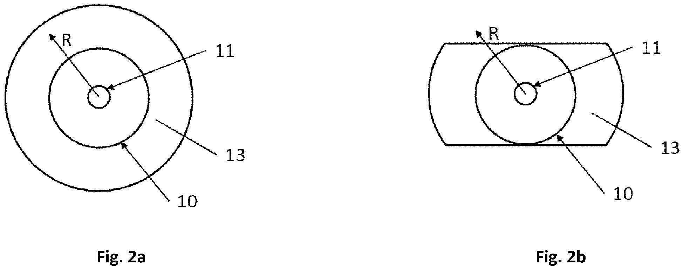

Fig. 2a zeigt eine schematische Draufsicht auf einen zylindrischen Untersuchungsbereich 13 einer Glasspritze 1 senkrecht zur Längsachse L, d.h. die Längsachse L geht mittig durch die Ansicht hindurch (nicht dargestellt). Von der Längsachse L geht senkrecht die radiale Richtung R ab. In der Mitte der Darstellung ist als innerer Kreis die zylindrische Bohrung 11, als nächstgrößerer mittlerer Kreis der Spritzenkörpers 10 und als nächstgrößerer Kreis die Fingerauflage 13 dargestellt. Fig. 2b zeigt eine entsprechende Darstellung mit einem zylindrisch unterbrochenen Untersuchungsbereich 13 einer Glasspritze 1 in Form einer abschnittsweise ausgebildeten Fingerauflage 13. Diese Form der Fingerauflage 13 kann auch als abgeschnittener Vollkreis betrachtet werden.

Derartige Fingerauflagen 13 werden herkömmlich wie folgt optisch untersucht:

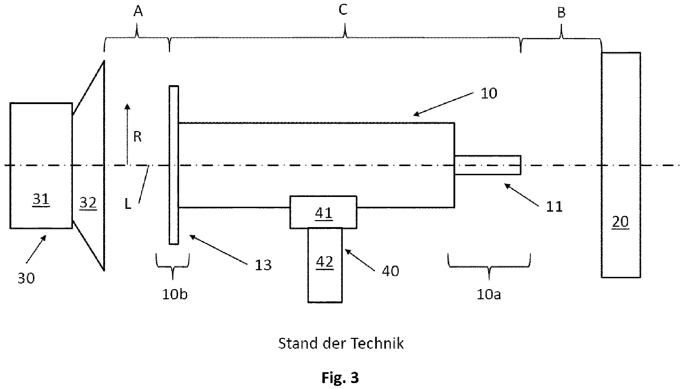

Fig. 3 zeigt eine schematische Schnittdarstellung durch ein Bilderfassungssystem für Glaskörper 1 in Form von Glasspritzen 1 entlang der Längsachse L gemäß dem Stand der Technik. Die Glasspritze 1 wird mit ihrem Spritzenkörpers 10 etwa mittig in Richtung der Längsachse L durch eine Handhabungseinheit 40 mittels deren Auflagefläche 41 von unten gehalten und in dem Bilderfassungssystem positioniert. Die Auflagefläche 41 wird über eine Halterung 42 gehalten und bewegt. Treten in einer Massenproduktion von Glasspritzen 1 die zu untersuchenden Objekte kontinuierlich durch das Bilderfassungssystem hindurch bzw. an diesem vorbei, so kann die zu untersuchende Glasspritze 1 z.B. von unten mittels der Handhabungseinheit 40 in das Bilderfassungssystem hinein angehoben und nach erfolgter Bilderfassung wieder zurück in den Fertigungsprozess abgelegt werden.

Auf der linken Seite der Fig. 3 ist eine optische Bilderfassungseinheit 30 angeordnet, welche eine Kameraeinheit 31 in Form einer Flächenkamera 31 aufweist. Die Flächenkamera 31 weist ein Objektiv 32 auf, mit dem sie auf die Fingerauflage 13 als Untersuchungsbereich 13 direkt hin ausgerichtet ist. Idealerweise wird die Glasspritze 1 durch die Handhabungseinheit 40 mit ihrer Längsachse L so im Bilderfassungssystem positioniert, dass ihre Längsachse L mit der entsprechenden Längsachse L des Objektivs 32 zusammenfällt. Das Objektiv 32 ist radial wenigstens etwas größer als der maximale Radius R der Fingerauflage 13, um diese als Untersuchungsbereich 13 sicher vollständig erfassen zu können. Das Objektiv 32 weißt gegenüber der Fingerauflage 13 einen Abstand A auf.

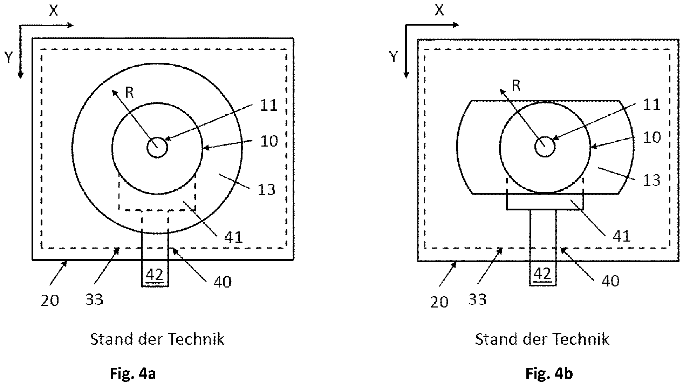

Auf der rechten Seite der Fig. 3 ist eine Beleuchtungseinheit 20 angeordnet, welche zur Bohrung 11 den Abstand B aufweist. Die Beleuchtungseinheit 20 ist entsprechend der zylindrischen Glasspritze 1 sowie des zylindrischen Objektivs 32 ebenfalls zylindrisch ausgebildet. Die Längsachse L der Beleuchtungseinheit 20 fällt mit der Längsachse L des Objektivs 32 zusammen. Die Beleuchtungseinheit 20 ist radial wenigstens etwas größer als der maximale Radius R des zu beleuchtenden Objekts, d.h. der Fingerauflage 13, um deren Untersuchungsbereich 13 (vgl. Fig. 4a, 4b) sicher vollständig ausleuchten zu können. Da auch bei sehr kleinen Glasspritzen 1 bzw. entsprechend kleinen Objektiven 32 die über diese radial hinausragende Beleuchtungseinheit 20 im Allgemeinen nicht durch eine einzige Lichtquelle gebildet werden kann, weist die Beleuchtungseinheit 20 mehrere einzelne Lichtquellen auf, welche gemeinsam die Beleuchtungseinheit 20 bilden.

Die durch die Handhabungseinheit 40 zwischen das Objektiv 32 und die Beleuchtungseinheit 20 positionierte Glasspritze 1 wird vom Licht der Beleuchtungseinheit 20 von rechts her durchleuchtet und ein Teil der Lichtstrahlen von der Glasspritze 1 her durch das Objektiv 32 an die Flächenkamera 31 geleitet, welche diese als flächiges Bild erfasst. Dabei ist das Objektiv 32 eingerichtet, für die Flächenkamera 31 einen Erfassungsbereich 33 zu erzeugen, der den Untersuchungsbereich 13 des Glaskörpers 1, d.h. deren Fingerauflage 13, vollständig erfasst aber irrelevante Bereiche um diesen Bereich herum ausschließt (vgl. Fig. 4a, 4b). Aufgrund der Größe der Beleuchtungseinheit 20 wird die vollständige Ausleuchtung des Erfassungsbereichs 33 der Flächenkamera 31 sichergestellt.

Fig. 4a zeigt eine schematische Darstellung eines flächigen Erfassungsbereichs 13 eines zylindrischen Untersuchungsbereichs 33 senkrecht zur Längsachse L gemäß dem Stand der Technik, d.h. gemäß dem System der Fig. 3. Fig. 4b zeigt eine entsprechende schematische Darstellung eines flächigen Erfassungsbereichs 13 eines zylindrisch unterbrochenen Untersuchungsbereichs 33. Neben den Objekten 10, 11, 13 der Glasspritze 1, wie bezüglich der Fig. 2a, 2b beschrieben, wird hier jeweils der rechteckige Erfassungsbereich 33 (gestrichelt) der Flächenkamera 31 dargestellt, welcher sich in X-Richtung und Y-Richtung erstreckt. Der Erfassungsbereich 33 erfasst die Fingerauflage 13 als Untersuchungsbereich 13 vollständig. Die Beleuchtungseinheit 20 bzw. der durch sie ausgeleuchtete Bereich erstreckt sich zumindest geringfügig über den Erfassungsbereich 33 in beiden Richtungen hinaus. Die Handhabungseinheit 40 bzw. ihrer Auflagefläche 41 und Halterung 42 sind zumindest teilweise durch die Fingerauflage 13 verdeckt (gestrichelt), weil sich diese aus der Richtung des Objektivs 32 hinter der Fingerauflage 13 und im Erfassungsbereich 33 befinden.

Aus den Darstellungen der Fig. 4a, 4b lassen sich die Nachteile des Bilderfassungssystems gemäß dem Stand der Technik erkennen. Zum einen wird die Handhabungseinheit 40 durch die Flächenkamera 31 stets mit erfasst, was die Bildverarbeitung in diesem Teilbereich des Untersuchungsbereichs 13 bei der Erkennung dimensionale und kosmetische Merkmale behindert. Ferner befindet sich die Beleuchtungseinheit 20 bezogen auf die Glasspritze 1 am weitesten von der Fingerauflage 13 entfernt, der jedoch erfasst und damit durch die Beleuchtungseinheit 20 ausgeleuchtet werden soll. Somit erreicht nur ein Teil des von der Beleuchtungseinheit 20 ausgesendeten Lichtes auch das Objektiv 32 der Flächenkamera 31. Außerdem befindet sich die Handhabungseinheit 40 zwischen der Beleuchtungseinheit 20 und der Flächenkamera 31, wodurch die Handhabungseinheit 40 einen Schatten im Erfassungsbereich 33 erzeugt.

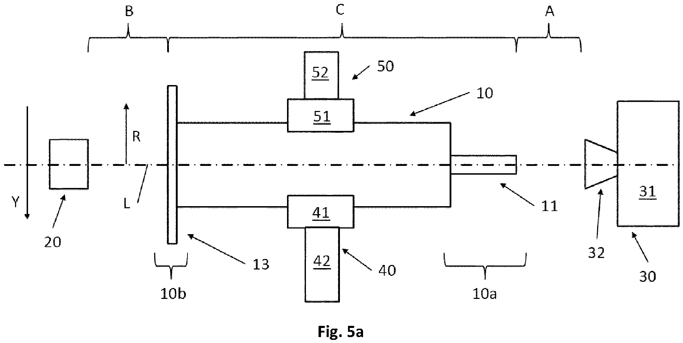

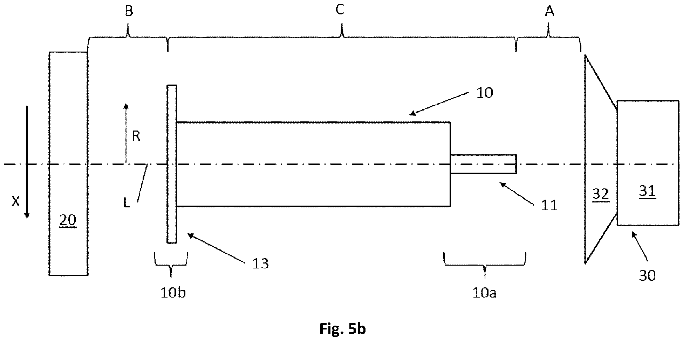

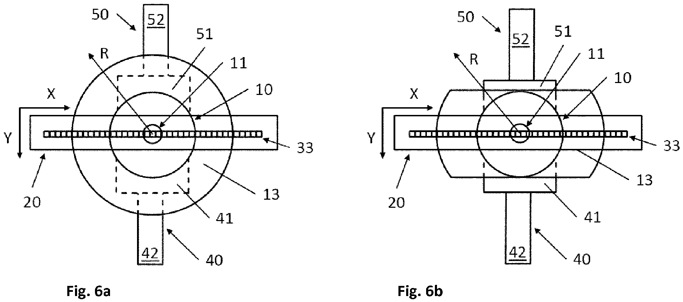

Fig. 5a zeigt eine schematische Schnittdarstellung durch ein erfindungsgemäßes Bilderfassungssystem für Glaskörper 1 in Form von Glasspritzen 1 entlang der Längsachse L von der Seite. Fig. 5b zeigt eine entsprechende Darstellung von Oben. Fig. 6a zeigt eine schematische Darstellung eines zeilenförmigen Erfassungsbereichs 33 eines zylindrischen Untersuchungsbereichs 13 senkrecht zur Längsachse L gemäß der vorliegenden Erfindung. Fig. 6b zeigt eine entsprechende schematische Darstellung eines zylindrisch unterbrochenen Untersuchungsbereichs 13.

Das erfindungsgemäße Bilderfassungssystem unterscheidet sich im Wesentlichen durch den Einsatz einer Zeilenkamera 31 anstelle einer Flächenkamera 31 von den bekannten Bilderfassungssystemen. Der Erfassungsbereich 33 einer Zeilenkamera 31 ist zeilenförmigen (vgl. Fig. 6a, 6b) und weist hierdurch eine höhere Auflösung auf als eine Zeile einer Flächenkamera 31. Dies alleine verbessert bereits die Qualität der Bilderfassung. Die Bildelemente dieser einen Zeile können eine Breite von nur 11 µm aufweisen. In der seitlichen Darstellung der Fig. 5a liegt die Zeile der Zeilenkamera 31 als Erfassungsbereich 33 auf der Längsachse L und ist aufgrund ihrer geringen Höhe nicht dargestellt (vgl. Fig. 6a, 6b). In der Draufsicht der Fig. 5b erstreckt sich die Zeile der Zeilenkamera 31 als Erfassungsbereich 33 über die Breite des Objektivs 32 (vgl. Fig. 6a, 6b).

Die Beleuchtungseinheit 20 ist entsprechend flach in der Höhe (vgl. Fig. 5a) aber breit in ihrer Breite (vgl. Fig. 5b) ausgebildet, so dass mit einer einzigen langgestreckten Lichtquelle bzw. einer Vielzahl von kleinen und seitlich eng benachbarten Lichtquellen (z.B. LEDs) eine Beleuchtungseinheit 20 mit einer (möglichst) durchgängigen und homogenen Lichtabstrahlung bereitgestellt werden kann. Diese überragt den einzeiligen Erfassungsbereich 33 sowohl in der Höhe als auch in der Breite zumindest derart geringfügig, dass der Erfassungsbereich 33 vollständig und möglichst homogen ausgeleuchtet ist, aber keine überflüssigen Lichtmengen in die angrenzenden Bereiche außerhalb des Erfassungsbereichs 33 abgegeben werden. Auch würden inhomogen angeordnete Lichtquellen außerhalb des Erfassungsbereichs 33 dennoch mit ihrem Licht auf den Erfassungsbereich 33 wirken und die homogene Lichtquelle bzw. homogenen Lichtquellen beeinträchtigen. Mit anderen Worten fallen Lichtdifferenzen durch die flächige Anordnung mehrerer Lichtquellen in der zweiten Richtung, d.h. der Höhe der Flächenkamera 31, durch den einzeiligen Erfassungsbereich 33 der Zeilenkamera 31 weg. Dies verbessert die Qualität der Ausleuchtung des Erfassungsbereichs 33 und damit auch die Qualität der Bilderfassung.

Eine weitere Verbesserung der Bilderfassung wird dadurch erreicht, dass die Anordnung von Bilderfassungseinheit 33 und Beleuchtungseinheit 20 gegenüber den bekannten Bilderfassungssystemen umgekehrt wird. Mit anderen Worten wird die Beleuchtungseinheit 20 direkt und möglichst nah, vorzugsweise in einem Abstand B von maximal 10 mm, direkt an der Fingerauflage 13 als Untersuchungsbereich 13 angeordnet. Die Bilderfassungseinheit 30 wird auf der gegenüberliegenden Seite der Glasspritze 1 angeordnet, nämlich auf dessen Bohrung 11 hin ausgerichtet, so dass die Zeilenkamera 31 die Fingerauflage 13 über die gesamte Glasspritze 1 hinweg und an der Bohrung 11 und dem Spritzenkörper 10 vorbei erfasst. Damit insbesondere die Ecken des Spritzenkörpers 10 am ersten, vorderen Ende die Fingerauflage 13 nicht verdecken, weist das Objektiv 32 eine entsprechend große Brennweite auf, die nahezu telezentrisch ist, so dass die Lichtstrahlen von der Fingerauflage 13 parallel zum Spritzenkörpers 10 auf das Objektiv 32 treffen. Hierdurch wird die äußerst nahe Positionierung der Beleuchtungseinheit 20 am Untersuchungsbereich 13 ermöglicht, so dass durch die Verbesserung der Ausleuchtung auch die Qualitäten der Bilderfassung der Fingerauflage 13 verbessert wird. Eine gleichzeitige Verschlechterung der Bilderfassung der Fingerauflage 13 durch die Zeilenkamera 31 kann durch die (nahezu) telezentrische Ausgestaltung des Objektivs 32 vermieden werden, so dass sich durch die Umkehr der üblichen Anordnung von Beleuchtungseinheit 20 und Bilderfassungseinheit 30 die Qualität der Bilderfassung weiter verbessern lässt.

Eine weitere wesentliche Verbesserung der Bilderfassung tritt dadurch ein, dass die Handhabungseinheit 40 und eine Bewegungseinheit 50 nicht durch die Zeilenkamera 31 erfasst werden und somit weder im erfassten Bild zu sehen sind noch die Ausleuchtung des Untersuchungsbereichs 13 behindern.

Die Bewegungseinheit 50 kann auch als Antriebseinheit 50 bezeichnet werden. Sie weist einen Bewegungsmechanismus 51 in Form einer Antriebsfläche 51 auf, die von oben durch eine Halterung 52 gehalten wird. Die Halterung 52 kann die Antriebsfläche 51 auch in der Höhe verfahren, um die Antriebsfläche 51 an eine zu erfassende Glasspritze 1 hereinzufahren und nach erfolgter Bilderfassung die Angriffsfläche 51 wieder von der Glasspritze 1 zu entfernen. Die Antriebsfläche 51 liegt in der Ausführungsform der Fig. 5a von oben auf dem Spritzenkörper 10 auf und kann hierdurch die Glasspritze 1 um ihre Längsachse L rotieren lassen, um den Untersuchungsbereich 13, d.h. die Fingerauflage 13, im Laufe der Drehung vollständig über den Erfassungsbereich 33 der Zeilenkamera 31 zu führen und hierdurch ein zweidimensionales Bild entstehen zu lassen.

Gleichzeitig liegt die Glasspritze 1 mit ihrem Spritzenkörpers 10 unten auf der Auflagefläche 41 der Handhabung zur Einheit 40 auf, die in diesem Fall als Rollfläche 41 ausgebildet ist, um mit der Antriebsfläche 51 zusammenwirken zu können. Alternativ kann auch die Bewegungseinheit 50 unterhalb und die Handhabungseinheit 40 oberhalb der Glasspritze 10 angeordnet sein.

### BEZUGSZEICHENLISTE (Teil der Beschreibung)

- A: Abstand zwischen Objektiv 32 und Glaskörper 1
- B: Abstand zwischen Beleuchtungsquelle 20 und Glaskörper 1
- C: Länge Glaskörper 1 von Bohrung 11 bis Fingerauflage 13
- L: Längsachse
- R: radiale Richtung
- X: X-Richtung eines erfassten Bildes
- Y: Y-Richtung eines erfassten Bildes

- 1: Glaskörper, Glasspritze
- 10: Spritzenkörper
- 10a: erstes, vorderes Ende des Spritzenkörpers 10
- 10b: zweites, hinteres Ende des Spritzenkörpers 10
- 11: Bohrung
- 12: Hohlnadel, Kanüle
- 13: zu erfassender Untersuchungsbereich des Glaskörpers 1, Fingerauflage der Glasspritze 1
- 14: Kolben

- 20: Beleuchtungseinheit, Lichtquelle

- 30: optische Bilderfassungseinheit
- 31: Kameraeinheit, Flächen- bzw. Zeilenkamera, Sensoreinheit
- 32: Objektiv
- 33: Erfassungsbereich, Bildpunkte, Bildpixel

- 40: Handhabungseinheit, Führungseinheit
- 41: Auflagefläche, Rollfläche
- 42: Halterung

- 50: Bewegungseinheit, Antriebseinheit
- 51: Bewegungsmechanismus, Antriebsfläche
- 52: Halterung

## Patentansprüche

1. System zur optischen Erfassung einer Fingerauflage (13) einer Glasspritze (1),
mit einer Glasspritze (1), welche entlang ihrer Längsachse (L) einen zylindrischen Spritzenkörper (10) mit einem vorderen Ende (10a) mit einer Bohrung (11) und mit einem hinteren Ende (10b) mit einer Fingerauflage (13) aufweist,
mit einer optischen Bilderfassungseinheit (30) zum Erfassen der Fingerauflage (13) der Glasspritze (1),
wobei die optische Bilderfassungseinheit (30) eine Zeilenkamera (31) mit einem zeilenförmigen Erfassungsbereich (33) aufweist, und
mit einer Bewegungseinheit (50), welche eingerichtet ist, die Glasspritze (1) und die Zeilenkamera (31) relativ zueinander derart zu bewegen, so dass die Fingerauflage (13) der Glasspritze (1) über den Erfassungsbereich (33) der Zeilenkamera (31), oder umgekehrt, geführt wird,
wobei
die optische Bilderfassungseinheit (30) eingerichtet ist, die Glasspritze (1) derart aufzunehmen, dass wenigstens der Spritzenkörper (10) der Glasspritze(1) entlang der Längsachse (L) zwischen der Fingerauflage (13) der Glasspritze (1) und der Bilderfassungseinheit (30) angeordnet ist, wobei die Bilderfassungseinheit (30) eingerichtet ist, die Fingerauflage (13) der Glasspritze (1) außen an dem Spritzenkörper (10) der Glasspritze (1) entlang der Längsachse (L) vorbei zu erfassen,
wobei die Bilderfassungseinheit (30) ein Objektiv (32) mit einer derart großen Brennweite aufweist, so dass Lichtstrahlen von der Fingerauflage (13) der Glasspritze (1) etwa parallel vom Objektiv (32) erfasst werden können,
wobei das Objektiv (32) eine telezentrische Brennweite oder eine hyperzentrische Brennweite aufweist.

2. System nach Anspruch 1,
wobei die Bewegungseinheit (50) eingerichtet ist, die Glasspritze (1) relativ zur Zeilenkamera (31), oder umgekehrt, derart um die Längsachse (L) zu drehen, so dass die Fingerauflage (13) der Glasspritze (1) über den Erfassungsbereich (33) der Zeilenkamera (31), oder umgekehrt, geführt wird.

3. System nach Anspruch 2,
wobei die Fingerauflage (13) der Glasspritze (1) oder der Erfassungsbereich (33) der Zeilenkamera (31) senkrecht zur Längsachse (L) ausgerichtet ist.

4. System nach einem der vorherigen Ansprüche,
ferner mit einer Beleuchtungseinheit (20) zur Ausleuchtung wenigstens der Fingerauflage (13) der Glasspritze (1).

5. System nach Anspruch 4,
wobei die Beleuchtungseinheit (20) einen geringeren Abstand (B) zur Fingerauflage (13) der Glasspritze (1) als die Bilderfassungseinheit (30) aufweist.

6. System nach Anspruch 4 oder 5,
wobei die Beleuchtungseinheit (20) einen Abstand (B) von weniger als 10 mm zur Fingerauflage (13) der Glasspritze (1) aufweist.

7. System nach einem der Ansprüche 4 bis 6,
wobei die Beleuchtungseinheit (20) eingerichtet ist, eine rechteckige Ausleuchtung der Fingerauflage (13) der Glasspritze (1) zu erzeugen, welche wenigstens dem Erfassungsbereich (33) der Zeilenkamera (31) entspricht.

8. System nach einem der vorherigen Ansprüche,
wobei die Bewegungseinheit (50) eine Antriebseinheit (51) aufweist, die eingerichtet ist, die Glasspritze (1) relativ zur Zeilenkamera (31), oder umgekehrt, zu bewegen.

9. System nach einem der vorherigen Ansprüche,
ferner mit einer Führungseinheit (40), welche eingerichtet ist, die Glasspritze (1) bei einer Relativbewegung zur Zeilenkamera (30), oder umgekehrt, zu führen.

10. Verfahren zur optischen Erfassung einer Fingerauflage (13) einer Glasspritze (1) mittels eines Systems nach einem der vorherigen Ansprüche,
wobei die Glasspritze (1) und die Zeilenkamera (31) relativ zueinander derart bewegt werden, so dass die Fingerauflage (13) der Glasspritze (1) über den Erfassungsbereich (33) der Zeilenkamera (31), oder umgekehrt, geführt wird,
wobei
die optische Bilderfassungseinheit (30) eingerichtet ist, den Glasspritze (1) derart aufzunehmen, dass wenigstens der Spritzenkörper (10) der Glasspritze (1) entlang der Längsachse (L) zwischen der Fingerauflage (13) der Glasspritze (1) und der Bilderfassungseinheit (30) angeordnet ist,
wobei die Bilderfassungseinheit (30) eingerichtet ist, die Fingerauflage (13) der Glasspritze (1) außen an dem Spritzenkörper (10) der Glasspritze (1) entlang der Längsachse (L) vorbei zu erfassen,
wobei die Bilderfassungseinheit (30) ein Objektiv (32) mit einer derart großen Brennweite aufweist, so dass Lichtstrahlen von der Fingerauflage (13) der Glasspritze (1) etwa parallel vom Objektiv (32) erfasst werden können,
wobei das Objektiv (32) eine telezentrische Brennweite oder eine hyperzentrische Brennweite aufweist.

## Claims

1. System for optically detecting a finger rest (13) of a glass syringe (1), comprising a glass syringe (1) which has, along its longitudinal axis (L), a cylindrical syringe body (10) having a front end (10a) with a bore (11) and having a rear end (10b) with a finger rest (13), comprising an optical image detection unit (30) for detecting the finger rest (13) of the glass syringe (1),
wherein the optical image detection unit (30) comprises a line camera (31) having a line-shaped detection region (33), and
comprising a movement unit (50) which is configured to move the glass syringe (1) and the line camera (31) relative to each other in such a way that the finger rest (13) of the glass syringe (1) is guided over the detection region (33) of the line camera (31), or vice versa, wherein
the optical image detection unit (30) is configured to receive the glass syringe (1) such that at least the syringe body (10) of the glass syringe (1) is arranged along the longitudinal axis (L) between the finger rest (13) of the glass syringe (1) and the image detection unit (30), wherein the image detection unit (30) is configured to detect the finger rest (13) of the glass syringe (1) past the outside of the syringe body (10) of the glass syringe (1) along the longitudinal axis (L),
wherein the image detection unit (30) has a lens (32) that has such a large focal length that light rays from the finger rest (13) of the glass syringe (1) can be detected approximately in parallel by the lens (32),
wherein the lens (32) has a telecentric focal length or a hypercentric focal length.

2. System according to claim 1,
wherein the movement unit (50) is configured to rotate the glass syringe (1) relative to the line camera (31), or vice versa, about the longitudinal axis (L) such that the finger rest (13) of the glass syringe (1) is guided over the detection region (33) of the line camera (31), or vice versa.

3. System according to claim 2, wherein the finger rest (13) of the glass syringe (1) or the detection region (33) of the line camera (31) is aligned perpendicular to the longitudinal axis (L).

4. System according to any of the preceding claims,
further comprising an illumination unit (20) for illuminating at least the finger rest (13) of the glass syringe (1).

5. System according to claim 4,
wherein the illumination unit (20) has a smaller distance (B) from the finger rest (13) of the glass syringe (1) than the image detection unit (30).

6. System according to either claim 4 or claim 5,
wherein the illumination unit (20) has a distance (B) of less than 10 mm from the finger rest (13) of the glass syringe (1).

7. System according to any of claims 4 to 6,
wherein the illumination unit (20) is configured to generate a rectangular illumination of the finger rest (13) of the glass syringe (1), which illumination corresponds at least to the detection region (33) of the line camera (31).

8. System according to any of the preceding claims,
wherein the movement unit (50) has a drive unit (51) which is configured to move the glass syringe (1) relative to the line camera (31), or vice versa.

9. System according to any of the preceding claims,
further comprising a guide unit (40) which is configured to guide the glass syringe (1) during a relative movement with respect to the line camera (30), or vice versa.

10. Method for optically detecting a finger rest (13) of a glass syringe (1) by means of a system according to any of the preceding claims,
wherein the glass syringe (1) and the line camera (31) are moved relative to each other such that the finger rest (13) of the glass syringe (1) is guided over the detection region (33) of the line camera (31), or vice versa,
wherein
the optical image detection unit (30) is configured to receive the glass syringe (1) such that at least the syringe body (10) of the glass syringe (1) is arranged along the longitudinal axis (L) between the finger rest (13) of the glass syringe (1) and the image detection unit (30),
wherein the image detection unit (30) is configured to detect the finger rest (13) of the glass syringe (1) past the outside of the syringe body (10) of the glass syringe (1) along the longitudinal axis (L),
wherein the image detection unit (30) has a lens (32) that has such a large focal length that light rays from the finger rest (13) of the glass syringe (1) can be detected approximately in parallel by the lens (32),
wherein the lens (32) has a telecentric focal length or a hypercentric focal length.

## Revendications

1. Système permettant la détection optique d'un support pour doigts (13) d'une seringue en verre (1), comportant une seringue en verre (1) qui présente, le long de son axe longitudinal (L), un corps de seringue (10) cylindrique comportant une extrémité avant (10a) comportant un alésage (11) et comportant une extrémité arrière (10b) comportant un support pour doigts (13), comportant une unité de détection d'image (30) optique permettant de détecter le support pour doigts (13) de la seringue en verre (1),
dans lequel l'unité de détection d'image (30) optique présente une caméra linéaire (31) comportant une zone de détection (33) linéaire, et
comportant une unité de déplacement (50) qui est configurée pour déplacer la seringue en verre (1) et la caméra linéaire (31) l'une par rapport à l'autre de telle sorte que le support pour doigts (13) de la seringue en verre (1) est guidé sur la zone de détection (33) de la caméra linéaire (31), ou inversement, dans lequel
l'unité de détection d'image (30) optique est configurée pour recevoir la seringue en verre (1) de telle sorte qu'au moins le corps de seringue (10) de la seringue en verre (1) est disposé le long de l'axe longitudinal (L), entre le support pour doigts (13) de la seringue en verre (1) et l'unité de détection d'image (30), dans lequel l'unité de détection d'image (30) est configurée pour détecter le support pour doigts (13) de la seringue en verre (1) en passant à l'extérieur du corps de seringue (10) de la seringue en verre (1), le long de l'axe longitudinal (L),
dans lequel l'unité de détection d'image (30) présente un objectif (32) comportant une grande distance focale de telle sorte que des rayons lumineux provenant du support pour doigts (13) de la seringue en verre (1) peuvent être détectés approximativement parallèlement à l'objectif (32),
dans lequel l'objectif (32) présente une distance focale télécentrique ou une distance focale hypercentrique.

2. Système selon la revendication 1,
dans lequel l'unité de déplacement (50) est configurée pour faire tourner la seringue en verre (1) par rapport à la caméra linéaire (31), ou inversement, autour de l'axe longitudinal (L) de telle sorte que le support pour doigts (13) de la seringue en verre (1) est guidé sur la zone de détection (33) de la caméra linéaire (31), ou inversement.

3. Système selon la revendication 2, dans lequel le support pour doigts (13) de la seringue en verre (1) ou la zone de détection (33) de la caméra linéaire (31) est orientée perpendiculairement à l'axe longitudinal (L).

4. Système selon l'une des revendications précédentes,
comportant en outre une unité d'éclairage (20) pour l'éclairage d'au moins le support pour doigts (13) de la seringue en verre (1).

5. Système selon la revendication 4,
dans lequel l'unité d'éclairage (20) présente une distance (B) par rapport au support pour doigts (13) de la seringue en verre (1) inférieure à celle entre l'unité de détection d'image (30) et ledit support pour doigts.

6. Système selon la revendication 4 ou 5,
dans lequel l'unité d'éclairage (20) présente une distance (B) de moins de 10 mm par rapport au support pour doigts (13) de la seringue en verre (1).

7. Système selon l'une des revendications 4 à 6,
dans lequel l'unité d'éclairage (20) est configurée pour produire un éclairage rectangulaire du support pour doigts (13) de la seringue en verre (1), lequel éclairage correspond au moins à la zone de détection (33) de la caméra linéaire (31).

8. Système selon l'une des revendications précédentes,
dans lequel l'unité de déplacement (50) présente une unité d'entraînement (51) qui est configurée pour déplacer la seringue en verre (1) par rapport à la caméra linéaire (31), ou inversement.

9. Système selon l'une des revendications précédentes,
comportant en outre une unité de guidage (40) qui est configurée pour guider la seringue en verre (1) dans un mouvement relatif par rapport à la caméra linéaire (30), ou inversement.

10. Procédé permettant la détection optique d'un support pour doigts (13) d'une seringue en verre (1) au moyen d'un système selon l'une des revendications précédentes,
dans lequel la seringue en verre (1) et la caméra linéaire (31) sont déplacées l'une par rapport à l'autre de telle sorte que le support pour doigts (13) de la seringue en verre (1) est guidé sur la zone de détection (33) de la caméra linéaire (31), ou inversement,
dans lequel
l'unité de détection d'image (30) optique est configurée pour recevoir la seringue en verre (1) de telle sorte qu'au moins le corps de seringue (10) de la seringue en verre (1) est disposé le long de l'axe longitudinal (L), entre le support pour doigts (13) de la seringue en verre (1) et l'unité de détection d'image (30),
dans lequel l'unité de détection d'image (30) est configurée pour détecter le support pour doigts (13) de la seringue en verre (1) en passant à l'extérieur du corps de seringue (10) de la seringue en verre (1), le long de l'axe longitudinal (L),
dans lequel l'unité de détection d'image (30) présente un objectif (32) comportant une grande distance focale de telle sorte que des rayons lumineux provenant du support pour doigts (13) de la seringue en verre (1) peuvent être détectés approximativement parallèlement à l'objectif (32),
dans lequel l'objectif (32) présente une distance focale télécentrique ou une distance focale hypercentrique.
